# EUROPÄISCHE PATENTANMELDUNG

(11) **EP 0 541 192 A1**
(43) Veröffentlichungstag der Anmeldung: **12.05.1993**
(21) Anmeldenummer: 92250317.2
(22) Anmeldetag: 02.11.1992
(51) Int. Cl.: A61K 31/485

(54) **Verwendung von Opiat-Antagonisten zur Behandlung von endogener Hyperinsulinämie**

(30) Priorität: 02.11.1991 DE 4136215
(71) Anmelder: Ferring Arzneimittel GmbH, D-24109 Kiel (DE)
(72) Erfinder: Leyendecker, G., Prof. Dr. med., W-6100 Darmstadt (DE); Wildt, Ludwig, Prof. Dr. med., 21-23, W-8520 Erlangen (DE)
(74) Vertreter: UEXKÜLL & STOLBERG

(57) **Zusammenfassung**

Die Erfindung betrifft die Verwendung von Opiat-Antagonisten zur Behandlung von endogner Hyperinsulinämie und durch diese induzierte Erkrankungen. Die verwendeten Wirkstoffe führen insbesondere eine schnelle Senkung des Körpergewichts ohne dietetische und sonstige restriktive Maßnahmen herbei.

## Beschreibung

Die Erfindung betrifft die Verwendung von Opiat-Antagonisten zur Behandlung von endogener Hyperinsulinämie und durch diese induzierte Erkrankungen, insbesondere von Hyperandrogenämie und Fettsucht (vgl. David E Moller and Jeffrey S. Flier in The New England Journal of Medicine 325, 938 - 948 (1991).

Insulinresistenz und deren zwangläufige Folge, Hyperinsulinämie, steht mit einer Reihe von Erkrankungen, wie Hyperandrogenämie, Hirsutismus und endometrialer Hyperplasie, in der Peri- und Postmenopause bei Frauen, jedoch auch mit beispielsweise essentiellem Bluthochdruck und Adipositas bei Männern und Frauen in engem Zusammenhang.

Im Bereich der Frauenheilkunde spielt die Verbindung von Hyperinsulinämie mit Hyperandrogenämie und Adipositas eine besondere Rolle. Die Ursachen und Zusammenhänge sind jedoch noch weitgehend ungeklärt (vgl. Leonid Poretsky "On the Paradox of Insulin-Inducded Hyperandrogenism in Insulin-Resistant States", Endocrine Reviews **12**, Seiten 3 bis 13).

Bisher stehen keine Medikamente zur Behandlung der Hyperinsulinämie zur Verfügung.

Es ist demgemäß Aufgabe der Erfindung, Mittel zur Behandlung von Hyperinsulinämie und verwandten Erkrankungen zu schaffen, die durch Eingriff auf der Ebene der diesen Erkrankungen zugrundeliegenden Insulinresistenz neue Therapiemöglichkeiten erschließen.

Zur Lösung dieser Aufgabe wird die Verwendung von Opiat-Antagonisten der allgemeinen Formel 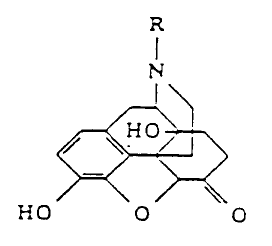
vorgeschlagen, worin R Allyl, Propargyl, 3-Methyl-2-butenyl, Cyclopropylmethyl oder Cyclobutylmethyl ist.

Ein erfindungsgemäß besonders bevorzugter Opiat-Antagonist ist Naltrexon (17-(Cyclopropylmethyl)-4,5-epoxy-3,14-dihydroxymorphinan-6-on) sowie Naloxon (17-Allyl-4,5-epoxy-3,14-dihydroxymorphinan-6-on).

Überraschenderweise hat es sich gezeigt, daß Opiat-Antagonisten der oben genannten Art und insbesondere Naltrexon bei Patientinnen mit Hyperandrogenämie assoziierter Hyperinsulinämie die Konzentration des als Reaktion auf Kohlenhydratbelastung im Serum zirkulierenden Insulins in überraschendem Umfang senkt. Die erfindungsgemäßen Befunde lassen darauf schließen, daß Opiat-Antagonisten die Sensibilität der Zielorgane gegenüber Insulin steigern.

Ferner weist die Erhöhung des molaren Verhältnisses von C-Peptiden zu Insulin (vgl. Beispiel 1) daraufhin, daß neben der Sensibilisierung der Zielorgane auch Änderungen im Insulinmetabolismus stattfinden, die eine erhöhte Clearance-Rate des Hormons zur Folge haben.

Ein besonders überraschender und vorteilhafter Effekt der erfindungsgemäß vorgeschlagenen Verwendung von Opiat-Antagonisten ist die schnelle Senkung des Körpergewichts, die ohne jede dietetische und restriktive Maßnahmen erzielt wird.

Die Wirkung der erfindungsgemäß verwendeten Opiat-Antagonisten im Zusammenhang mit der Behandlung der Hyperinsulinämie eröffnet somit nicht nur Möglichkeiten zur Therapie der Hyperandrogenämie und den mit dieser Erkrankung verbundenen klinischen Erscheinungsbildern, sondern sie ermöglicht auch die Behandlung und Prophylaxe von Hyperinsulinämie bedingter Adipositas und essentiellem Bluthochdruck bei Männern und Frauen.

Schließlich läßt sich auch Altersdiabetes durch die erfindungsgemäß vorgeschlagene Verwendung von Opiat-Antagonisten günstig beeinflussen.

Die erfindungsgemäß verwendeten Opiat-Antagonisten und ihre Herstellung werden in der GB-PS 939 287 und der US-PS 3 332 950 beschrieben. Es handelt sich um Antagonisten für Narkotika bzw. Opiate, deren Hauptverwendungszweck in der postoperativen Verabreichung nach der Verwendung von Opiat-Analgetika bestand. Wie in der DE-PS 36 31 088 beschrieben, können sie ferner zur Diagnose und Therapie von durch Endorphin bedingten Störungen der Sekretion von GnRH und Gonadotropien eingesetzt werden.

Die erfindungsgemäß verwendeten Opiat-Antagonisten können oral und/oder parenteral und/oder transdermal verabreicht werden, wobei Naloxon eine relativ kurze Halbwertszeit hat und daher die parenterale Applikation, beispielsweise als Dauerinfusion, bevorzugt ist. Demgegenüber wird Naltrexon, welches eine lange Verweildauer im Plasma aufweist, vorzugsweise peroral oder transdermal verabreicht.

Zur Applikation werden die Opiat-Antagonisten mit üblichen Hilfs- und Trägerstoffen für die jeweilige Verabreichungsform formuliert. Die Verabreichungsdauer und die Dosis der jeweils verwendeten Substanz richten sich nach dem Ausmaß der zu therapierenden Funktionsstörung, wobei beispielsweise bei Hyperandrogenämie und/oder Adipositas von einer Langzeitapplikation auszugehen ist. Für Naltrexon kommen in diesem Zusammenhang tägliche Dosen in der Größenordnung von 50 mg in Betracht.

Die Erfindung wird nachfolgend anhand eines Beispiels erläutert.

### Beispiel

Die folgenden Untersuchungen wurden an vier gegenüber Insulin resistenten Frauen durchgeführt, welche ein Hyperandrogenämie litten. Das Alter der Frauen lag zwischen 16 und 33 Jahren und ihr Körpermasseindex (Body mass Index - BMI) reichte von 28 bis 41. Die Hyperandrogenämie wurde durch Bestimmung von LH, FSH, Prolactin, Testosteron, freien Testosteronen, SHBG und DHEAS im Serum diagnostiziert. Vor der Behandlung lagen die Testosteronwerte zwischen 0,4 und 1,65 ng/ml und die Insulinkonzentrationen betrugen von 25 bis 62 mcU/ml.

### Nachweis der Insulinresistenz:

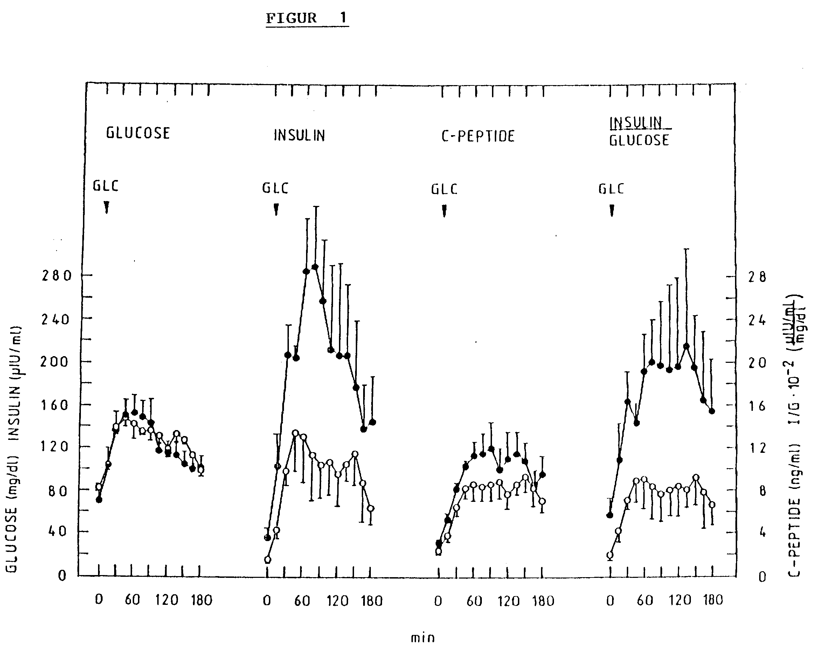

Die Insulinresistenz wurde durch orale Verabreichung von 400 ml einer Lösung nachgewiesen, welche eine Mono-Oligosaccharidmischung enthielt, die nach enzymatischer Hydrolyse im Gastrointestinaltrakt 100 g Glucose lieferte (Dextro-oGTT, Boehringer Mannheim). Während des oralen Glucose-Toleranz-Tests (oGTT) wurden vor der Kohlenhydratbelastung und danach 3 Stunden lang im Abstand von jeweils 15 Minuten Blutproben entnommen. In allen Proben wurde die Glucosekonzentration mit dem Hexokinase/Glucose-6-Phosphatdehydrogenase-Verfahren bestimmt. Ferner wurden die Insulin- und C-Peptidkonzentrationen mit handelsüblichen Radioimmun-Kits (Serono Diagnostic, Freiburg) bestimmt. Die Ergebnisse des oGTT wurden ausgewertet, indem das Verhältnis von Insulin zu Glucose berechnet wurde und die Flächen unter den Glucose-, Insulin- und C-Peptid-Kurven während des oGTT bestimmt wurden (vgl. Figur 1).

### Behandlung:

Als Opiat-Antagonist wurde Naltrexon (Ferring Arzneimittel GmbH, Kiel) in einer Dosis von 50 mg/Tag oral verabreicht. 4 Wochen nach Beginn der Naltrexon-Behandlung wurde nach dem gleichen Verfahren wie oben beschrieben ein zweiter oGTT durchgeführt und die Ergebnisse dieses Tests mit den vor Beginn der Behandlung erhaltenen Ergebnissen verglichen, wobei die Proben paarweise einem t-Test unterworfen wurden.

### Ergebnisse:

Wie Figur 1 zeigt, stiegen die Glucose-Spiegel als Antwort auf die Kohlenhydratbelastung, blieben jedoch bei allen Patienten innerhalb der Normgrenzen. Im Gegensatz dazu gingen die Konzentrationen des zirkulierenden Insulins weit über diejenigen Spiegel hinaus, die bei nicht-hirsuten, nicht-hyperandrogenen Kontrollpatienten beobachtet wurden. Das Ergebnis war ein gesteigertes I/G-Verhältnis. Unter der Behandlung mit Naltrexon konnte jedoch eine beeindruckende Senkung des Insulinspiegels nach Kohlenhydratbelastung beobachtet werden.

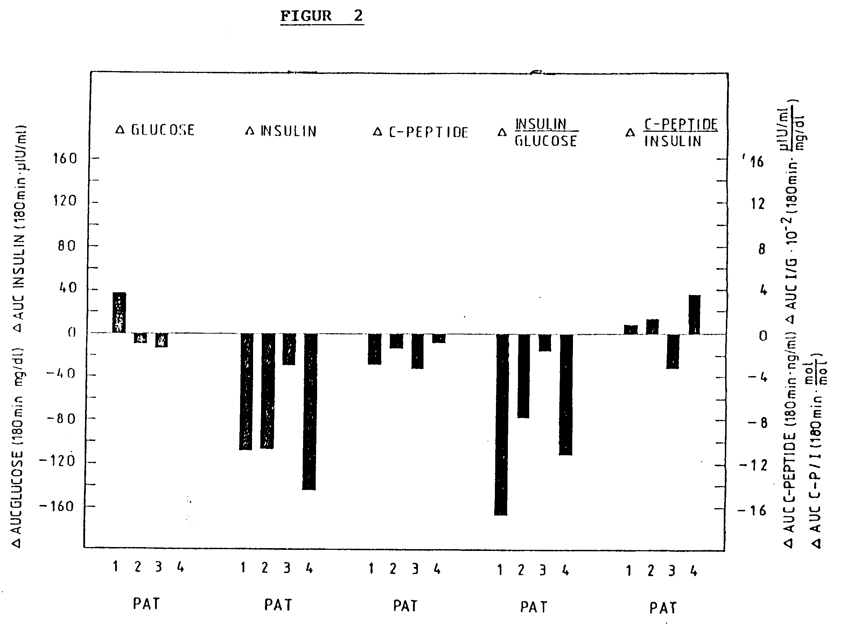

Die Konzentration von C-Peptid war während der Behandlung ebenfalls reduziert, jedoch nicht in gleichem Umfang wie von Insulin. Folglich sank das Verhältnis von Insulin zu Glucose während der Behandlung, das molare Verhältnis von C-Peptid zu Insulin stieg jedoch zur gleichen Zeit. Die Änderungen der Kurvenflächen (AUC) von Glucose, Insulin und C-Peptiden sowie das Verhältnis von Insulin zu Glucose und von C-Peptiden zu Insulin für jeden der Patienten sind in Figur 2 wiedergegeben.

Als besonders auffallendes Ergebnis der Naltrexon-Behandlung wurde bei den betroffenen Patienten ein erheblicher Gewichtsverlust beobachtet, welcher bezogen auf den gesamten kurzen Beobachtungszeitraum von 2 bis 9 kg reichte. Die Patienten berichteten übereinstimmend, daß dieser Gewichtsverlust ohne jede Diät oder sonstige Bemühungen um Gewichtsreduktion erreicht wurde.

Im Gegensatz dazu blieben die Androgenkonzentrationen sowie die LH-, FSH- und Prolactin-Spiegel durch die Naltrexon-Behandlung unbeeinflußt.

### Nebenwirkungen:

Das Naltrexon wurde von drei Patienten gut vertragen, während ein Patient während der Behandlung an Übelkeit mit Erbrechen und leichten Kopfschmerzen litt. Veränderungen des Blutbildes, der Leberenzyme und des Nierenstatus wurden nicht beobachtet.

## Patentansprüche

1. Verwendung von Opiat-Antagonisten der allgemeinen Formel 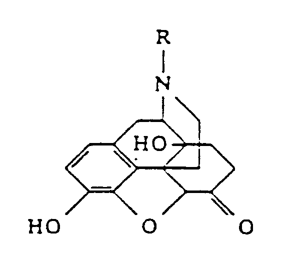 worin R Allyl, Propargyl, 3-Methyl-2-butenyl, Cyclopropylmethyl oder Cyclobutylmethyl ist, zur Herstellung eines Arzneimittels zur Behandlung von endogener Hyperinsulinämie sowie durch diese bedingte Erkrankungen.

2. Verwendung nach Anspruch 1, **dadurch gekennzeichnet,** daß R Allyl ist (Naloxon).

3. Verwendung nach Anspruch 2, **dadurch gekennzeichnet,** daß das Arzneimittel in Form einer parenteral zu verabreichenden Formulierung vorliegt.

4. Verwendung nach Anspruch 1, **dadurch gekennzeichnet,** daß R Cyclopropylmethyl ist (Naltrexon).

5. Verwendung nach Anspruch 4, **dadurch gekennzeichnet,** daß das Arzneimittel in Form einer peroral oder transdermal zu verabreichenden Formulierung vorliegt.

6. Verwendung nach einem der Ansprüche 1 bis 5 zur Herstellung eines Arzneimittels zur Behandlung von Hyperandrogenämie.

7. Verwendung nach einem der Ansprüche 1 bis 6 zur Herstellung eines Arzneimittels zur Behandlung von Adipositas.
